# EUROPEAN PATENT APPLICATION

(11) **EP 3 570 008 A1**
(43) Date of publication of application: **20.11.2019**
(21) Application number: 18172084.8
(22) Date of filing: 14.05.2018
(51) Int. Cl.: G01N 21/39, G01N 21/3504, G01N 21/359

(54) **NITROGEN TRICHLORIDE ANALYSER**

(71) Applicant: Universite Libre de Bruxelles, 1050 Bruxelles (BE)
(72) Inventor: FÖLDES, Tomas, 1050 Bruxelles (BE)
(74) Representative: Pronovem

(57) **Abstract**

The present invention is related to a device for continuously measuring nitrogen trichloride concentration in a gas comprising a gas inlet (2), a first filter (3) downstream of the gas inlet (2) impregnated with a reducing agent converting nitrogen trichloride into ammonia, means (4) for measuring ammonia concentration in the gas phase downstream of the filter (3) and a pump (7) for maintaining a flow through the filter (3).

## Description

### Field of the invention

The present invention is related to a real time nitrogen trichloride analyser.

### State of the art

Nitrogen trichloride is a volatile, irritant compound of penetrating odour, which is found as a disinfection by-product (DBP) in the air of chlorinated indoor swimming pools from reactions of nitrogenous compounds with chlorine. In swimming pool, such nitrogenous product are generated by the swimmers, in the form of sudation products, urea, ....

As reported in a draft version of the "Guidance for Biocidal Product Regulation: Volume V Disinfection By-Products", (PUBLIC Version 1.0 October 2016, published by the European Chemicals Agency), Nitrogen trichloride is strongly irritating for airways and available literature clearly indicates this DBP to be associated with adverse respiratory effects in swimmers and pool attendants in indoor swimming pools. By comparing measured air concentrations with an appropriate existing limit value in air the potential risk for local inhalation effects can be evaluated. In France a maximum of 500 µg/m3 has been in use from 1995 onwards (Hery et al. 1995) but ANSES and INRS now use a lower value of 300 µg/m3 (ANSES 2012). RIVM (2014) proposed using the 500 µg/m3 as maximum with a target value of 200 µg/m3. These values are all based on epidemiological surveys in which concentrations of nitrogen trichloride measured in swimming-pool air were correlated with respiratory health complaints among pool workers.

Nowadays, sampling and analysis of nitrogen trichloride (*NCl₃*) is generally performed using a method developed by Hery et al, described in article "Exposure to chloramines in the atmosphere of indoor swimming pools." Ann Occup Hyg (1995) 39 (4): 427-439 and is based on two successive chemical reactions:
1. at high pH, nitrogen trichloride is decomposed into ammonia and hypochlorite
   (1)

      *Na*₂*CO*₃ + *H*₂*O* → 2*Na*⁺ + *HCO*₃⁻ + *OH*⁻,
   (2)

      *NCl*₃ + 3*OH*⁻ → *NH*₃ + 3*ClO*⁻;
2. the hypochlorite ion is reduced to chloride by trivalent arsenic
   (3)

      *As*₂*O*₃ + 2*ClO*⁻ → *As*₂*O*₅ + *Cl*₂*.*
The principle of the method is consequently based on the sampling of nitrogen trichloride on a specific filter soaked with a solution of sodium carbonate (*Na₂CO₃*), diarsenic trioxide (*As₂O₃*) and glycerol (to trap the chloride). Typically, 37-mm cellulose support filters and few liters/minute flow vacuum pumps are used to sample swimming pool air. After sampling, the impregnated filters are desorbed and analyzed by a standard off-line technique (ion chromatography). Another reactive method uses an impinger system and the colorimetric chlorine test reagent N,N-diethyl-p-phenylenediamine (DPD) with potassium iodide.

It appears that the reaction scheme described by Hery et Al. is not correct and equation (2) has to be replaced by the hereafter reaction equation (4):
(4)

   2*NCl*₃ + 6*OH*⁻ → *N*₂ + 3*OCl*⁻ + 3*Cl*⁻ + 3*H*₂*O*
This change does not modify the prior art results, as in this analytical process, only chlorinated derivatives are measured.

The main drawback of the prior art analytical methods is that they are time consuming manual and off-line methods, well adapted for legal assessment of the compliance of installation to the legal limit, but not adapted to online monitoring and control of air quality.

In controlling swimming pool air quality, there is a need to strike a balance between conflicting parameters. On one hand, for improving air quality, it is desirable to increase air renewal by increasing ventilation. On the other hand, there is a need to decrease energy consumption and heat loss, which tends to decrease ventilation. The best compromise can only be obtained by online monitoring and loop-back control of the ventilation. So, there is a need for a device and a method for online monitoring of nitrogen trichloride in swimming-pool confined atmosphere.

### Summary of the invention

The present invention discloses a device for continuously measuring nitrogen trichloride concentration in a gas comprising an gas inlet, a first filter downstream of the gas inlet impregnated or coated with a reducing agent converting nitrogen trichloride into ammonia, means for measuring ammonia concentration in the gas phase downstream of the filter and a pump for maintaining a flow through the filter.

Preferred embodiments of the present invention disclose at least one, or an appropriate combination of the following features:
- the means for measuring ammonia concentration comprises a spectrophotometer, preferably a laser cavity ring-down spectrometer, more preferably, a continuous wave laser cavity ring-down spectrometer;
- the spectrophotometer is operated at near infra-red wavelength comprised between 780 nm and 2500 nm preferably between 1520 and 1535 nm;
- the reducing agent is a sulfite or a cyanide salt of a metal such as Na or K;
- a by-pass is located between the gas inlet and the means for measuring ammonia, for measuring the ammonia concentration in the gas without passing through the first filter, for measuring a background signal;
- a second filter is located upstream of the first filter said second filter comprising an ammonia trapping agent for removing ammonia from the gas to be analysed before conversion of the nitrogen trichloride, thereby reducing the background signal, said second filter comprising preferably a silica gel impregnated by sulfamic acid.

A second aspect of the invention is related to a method for analysing nitrogen trichloride in a gas atmosphere comprising the steps of:
- sampling the gas to be analysed;
- converting the nitrogen trichloride comprised in the sampled gas into ammonia and chlorinated by products;
- measuring ammonia concentration in the converted sampled gas;
- converting the measured ammonia concentration into an equivalent nitrogen trichloride concentration in the sampled gas.

Advantageously, the method of the invention comprises the additional step of removing the ammonia in the sampled gas before converting nitrogen trichloride into ammonia.

Preferably, the method comprises the step of measuring a background of the measured ammonia concentration by additionally measuring an ammonia concentration before the nitrogen trichloride conversion.

Advantageously, the sampling is a continuous sampling, and the measurement is performed in real time. By "continuous sampling", and "real time measurement", it is meant that there is a continuous flow of gas in the gas inlet, and the converted ammonia concentration is continuously monitored online. This means that the measurement is made without off-line measurement in a separate lab, the delay for obtaining the result being short enough to enable online regulation of the air renewal in the swimming pool building for regulating nitrogen trichloride concentration below acceptable level.

### Short description of the drawings

Fig. 1 represents a device according to the invention.
Fig. 2 represents measurement results of the example.

### List of reference symbols

1. inflow of sampled gas
2. inlet
3. first filter (conversion filter)
4. ammonia gas phase analyzer
5. outlet tube
6. exhaust gas
7. air pump
8. second filter (ammonia absorber)
9. by-pass
10. direct flow valve
11. by-pass valve

### Detailed description of the invention

Unfortunately, sensitivity of gas phase spectrometers are usually not sufficient for detecting nitrogen trichloride at concentration levels close to the 200µg/m³ limit usually accepted, so that off-line detection is usually used. As matter of fact, it was discovered by the applicant that gas phase ammonia has usually much lower detection limits, and more particularly detection limits below the ammonia concentration equivalent to 200µg/m³ nitrogen trichloride.

So the applicant first tried to convert the gaseous amine content of nitrogen trichloride into ammonia by reaction (2) as given by prior art articles. Unfortunately, this chemical equation was not verified, and conversion of nitrogen trichloride to ammonia and chlorinated by-products was not observed experimentally.

After some trials and errors, it was discovered that filters soaked in sodium sulfite (Na₂SO₃) were able to fully convert nitrogen trichloride to ammonia and chlorinated by-products, without producing noticeable amount of N₂, according to the following chemical scheme: 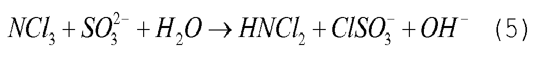 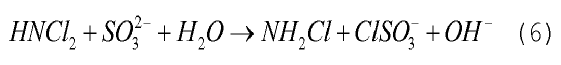 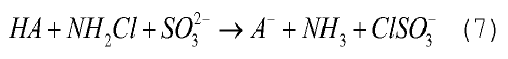 Where HA is an acid.

Surprisingly, the reaction was proven to fully occur on the surface of a filter coated (or impregnated) by dipping the filter in a concentrated sodium sulfite aqueous solution, even dried, atmospheric humidity (more particularly in wet atmosphere, such as in an indoor swimming pool) being sufficient to provide the H₂O involved in the reaction scheme. Therefore, this gas to gas conversion was adequate to make quantitative online measurements.

Online nitrogen trichloride monitoring is particularily useful regulate swimming pool ventilation systems that are, in prior art, either non-controlled or controlled based on other parameters, such as humidity or CO2 concentration.

Furthermore, a portable online monitoring system can also be used to detect eventual nitrogen trichloride accumulation zones.

In order to make online quantitative measurements according to the invention, a continuous flow of the gas to be analysed 1 is pumped through a first filter 3 previously impregnated by a sodium sulfite aqueous solution. Preferably, the filter is dried before use. In this process, the nitrogen trichloride gas content is almost fully converted to gaseous ammonia and chlorinated by-products.

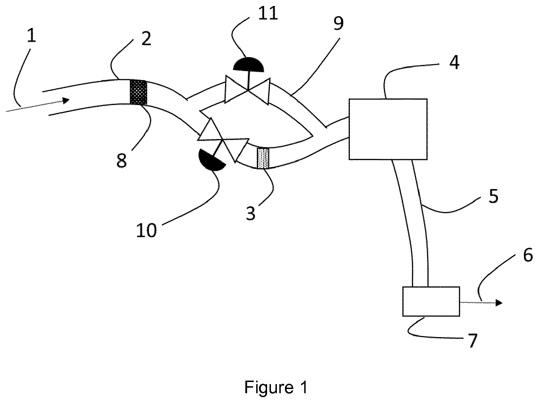

As seen in fig. 1, the gas downstream of the first filter, containing the converted ammonia is then passed through an analyser 4 able to detect ppb of ammonia (40ppb of nitrogen trichloride roughly corresponds to the target limit of 200µg/m³).

This analyser is preferably a near infra-red absorption spectrometer, measuring the absorbance of ammonia between 6548 and 6549 cm⁻¹. Other regions of the IR spectrum can be used, but the given range is the one leading to the best signal to noise ratio, and to the best sensitivity.

Preferably, the spectrometer measuring the near Infra-Red absorbance of the converted gas is a laser cavity ring-down spectrometer, most preferably of the continuous wave type, as those spectrometers are those having the lowest detection limit at an affordable price.

The air pump 7, forcing the gas flow through the filter is preferably located downstream of the analyser. The pump flow is chosen in orderto have sufficient contact time between the conversion filter 3 and the analysed gas 1, optimise response time of the system but also in order to increase the lifetime of the conversion filter 3.

Advantageously, as represented in fig. 1, a by-pass 9 controlled by a valve 11 permits to measure the signal without nitrogen trichloride conversion. This is useful for measuring a background signal, thereby improving the measurement precision. In that case, when measuring the signal, the valve 10 is opened and the by-pass valve 11 is closed, and when the background is measured, the valve 10 is closed and the by-pass valve 11 is opened.

Preferably, a second filter 8 is added on the inflow path for absorbing eventual ammonia present in the gas to be analysed before nitrogen trichloride conversion, in order to ensure that all the measured ammonia is indeed originating from the nitrogen trichloride present in the gas to be analysed. For example, this second filter may comprise silica gel impregnated by sulfamic acid which efficiently traps ammonia.

### Example

A two hour measurement has been performed at an indoor swimming pool.

The measurement was conducted using a near-infrared continuous wave cavity ring-down spectrometer built-in-house. The sample inlet of the spectrometer comprised of two air monitoring cassettes, a 3-way solenoid valve (to allow switching between the monitoring cassettes) and a borosilicate glass needle valve with a PTFE piston (to enable choking the flow).

All components were connected using PFA tubing (1/8" OD x 0.030" wall) between each other and the spectrometer. Vacuum was provided by a 5 SLPM (standard litre per minute) diaphragm vacuum pump downstream of the measurement cell of the spectrometer. The needle valve was adjusted so that the pressure in the spectrometer was about 80 mbar, leading to an actual flow rate of 0.4l/min of air through the selected monitoring cassette (5 SLPM (standard litre per minute) x 0.080 bar=0.4l/min).

The monitoring cassette housing a 37 mm hydrophobic PTFE membrane filter with 1.0 um pore size, to keep aerosols out of the measurement system, was used for background ammonia measurement.

The second monitoring cassette housed besides the PTFE membrane a 37 mm hydrophilic quartz fibre filter (thickness: 430 µm) that has been soaked in a sodium sulfite solution (31.5g/l of Na₂SO₃ yielding 20g/l of SO₃²⁻) and dried.

The cavity ring-down spectrometer was set to record the spectral range between 6548.5 and 6549cm⁻¹ in a loop. This spectral range contains 2 strong ammonia peaks. These peaks in the recorded spectra were fitted by Voight profiles in real-time by a least-squares fit algorithm. Relying on literature data (line intensity, broadening coefficients) the molar concentration of ammonia was determined from the area of the peaks and the pressure measurement in the measurement cell using a compact absolute capacitance manometer (full scale 133 milibar), and plotted on a chart. The scan of one spectrum took about 1 minute.

Air was sequentially sampled directly from the swimming pool atmosphere (i. e. through the by-pass 9, only aerosols being removed by a Teflon filter, not represented) and through the sodium sulfite soaked filter. The concentration of NH3 in the sampled gas was measured on-line. When sampling directly, only the concentration of NH3 in the swimming pool air is measured; when sampling through the chemical conversion filter, NCl3 in the air is transformed to NH3, and the measured concentration corresponds to the sum of NH3+NCl3. Hence the difference between the 2 measured concentrations corresponds to NCl3 concentration in the swimming pool air.

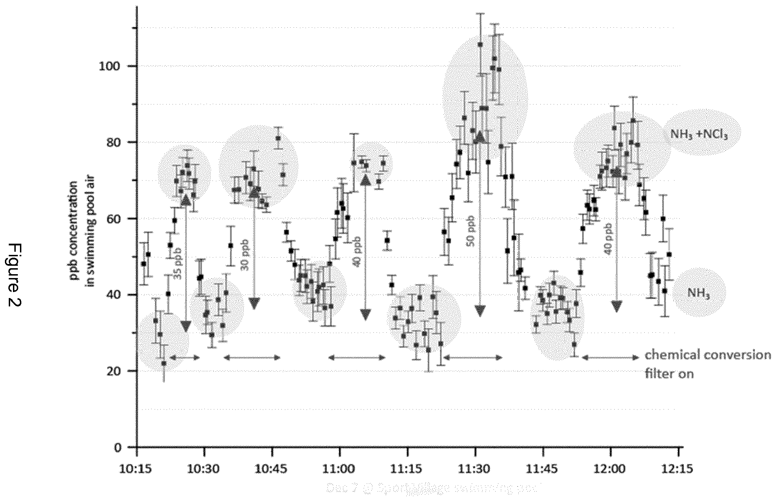

The obtained results are shown in Fig. 2. The conversion factor for NCl₃ between ppb (molar fraction of ammonia) and mg/m³ (the unit used in the regulation) is 0.005 mg/m³/ppb i.e. 40 ppb measured molar fraction of ammonia corresponds to 0.2 mg/m³ of nitrogen trichloride in the analysed gas.

Fig. 2 shows response time of the device of the invention of about 5 min. This response time is usually sufficient for example for controlling ventilation, nevertheless, it can even be shortened by using shorter tubing and higher flow rates if needed.

## Claims

1. Device for continuously measuring nitrogen trichloride concentration in a gas comprising a gas inlet (2), a first filter (3) downstream of the gas inlet (2) impregnated with a reducing agent converting nitrogen trichloride into ammonia, means (4) for measuring ammonia concentration in the gas phase downstream of the filter (3) and a pump (7) for maintaining a flow through the filter (3).

2. Device according to claim 1 wherein the means for measuring ammonia concentration comprises a spectrophotometer.

3. Device according to claim 2 wherein the spectrophotometer is a laser cavity ring-down spectrometer.

4. Device according to claim 3 wherein the laser cavity ring-down spectrometer is a continuous wave laser cavity ring-down spectrometer.

5. The device according to claim 2 or 3 wherein the spectrophotometer is operated at near infra-red wavelength comprised between 780 nm and 2500 nm preferably between 1520 and 1535 nm

6. Device according to any of the previous claims wherein the reducing agent is a sulfite or a cyanide salt of a metal such as Na or K.

7. Device according to any of the previous claims wherein a by-pass (9) is located between the gas inlet and the means (4) for measuring ammonia, for measuring the ammonia concentration in the gas without passing through the first filter, for measuring a background signal.

8. Device according to any of the previous claims comprising a second filter (8) upstream of the first filter (4), said second filter comprising an ammonia trapping agent for removing ammonia from the gas to be analysed before conversion of the nitrogen trichloride, thereby reducing the background signal.

9. Device according to claim 8 wherein the second filter comprises silica gel impregnated by sulfamic acid.

10. Method for analysing nitrogen trichloride in a gas atmosphere comprising the steps of:
- sampling the gas to be analysed;
- converting the nitrogen trichloride comprised in the sampled gas into ammonia and chlorinated by products;
- measuring ammonia concentration in the converted sampled gas;
- converting the measured ammonia concentration into an equivalent nitrogen trichloride concentration in the sampled gas.

11. Method according to claim 10 comprising the step of removing the ammonia in the sampled gas before converting nitrogen trichloride into ammonia.

12. Method according to claim 10 or 11 comprising the step of measuring a background of the measured ammonia concentration by additionally measuring an ammonia concentration before the nitrogen trichloride conversion.

13. Method according to any of the claims 10 to 12 wherein the sampling is a continuous sampling, and the measurement is performed in real time.
